Europäisches Patentamt

European Patent Office

Office européen des brevets

(11) Publication number: **0 268 421**
**A2**

# (12) EUROPEAN PATENT APPLICATION

(21) Application number: 87309974.1

(22) Date of filing: 11.11.87

(51) Int. Cl.⁴: **A61L 27/00** , **C08L 89/06**

(30) Priority: 17.11.86 JP 273156/86

(43) Date of publication of application:
**25.05.88 Bulletin 88/21**

(84) Designated Contracting States:
**DE FR GB IT NL SE**

(71) Applicant: **KOKEN CO. LTD.**
**5-18 Shimoochiai 3-chome**
**Shinjuku-ku Tokyo(JP)**

(72) Inventor: **Yahagi, Tsuyoshi Koken Co.,Ltd.**
**Japan Biomedical Material Research Center**
**2-11-21 Nakane Meguro-ku Tokyo(JP)**
Inventor: **Miyata, Teruo Koken Co.,Ltd.**
**Japan Biomedical Material Research Center**
**2-11-21 Nakane Meguro-ku Tokyo(JP)**

(74) Representative: **Marlow, Nicholas Simon et al**
**Reddie & Grose 16, Theobalds Road**
**London WC1X 8PL(GB)**

(54) An implant composition and a method of preparing the same.

㊸ An implant composition comprising an aqueous dispersion of atelocollagen crosslinked with hexamethylene diisocyanate has a high degree of volume retention and low immunogenicity in a living body, and is not cytotoxic.

EP 0 268 421 A2

0 268 421

## AN IMPLANT COMPOSITION AND A METHOD OF PREPARING THE SAME

This invention relates to an implant composition and to a method of preparing the same.

Collagen - the important protein which constitutes the connective tissues such as the skin, blood vessels, cornea, tendons, bones and teeth of animals - is biochemically synthesized by collagen-producing cells in a living body, and the synthesized molecules of the collagen are incorporated in the surrounding collagen fiber tissues, and gradually change into insoluble collagen throguh intermolecular crosslinking. The insoluble collagen cannot be extracted by dilute aqueous acid solutions. But, if treated with an proteolytic enzyme such as pepsine, the insoluble collagen undergoes fission at the intermolecular crosslinks and becomes soluble in dilute acids. During this treatment with pepsine, the telopeptide groups at both terminals of each collagen molecule are digested, leaving collagen with no telopeptide terminal end. The soluble collagen thus formed is called atelocollagen. Since the telopeptide groups at terminal ends is primarily responsible for antigenicity of collagen, atelocollagen has little antigenicity, which makes it very suitable for use as a medical material.

It has been proposed to inject atelocollagen as an implant material, in the form of an aqueous dispersion, into defective tissues to restore the affected part without cutting out the surrounding tissues. An improved technique has also been proposed in which atelocollagen previously crosslinked primarily with glutaraldehyde is used to lower immunogenicity and to ensure higher resistance to water absorption ( that is, higher degree of volume retention ) [ Japanese Patent Kokai No.170796 / 1983 and Japanese Patent Publication No.54288 / 1985 ]. It was reported that atelocollagen crosslinked with glutaraldehyde was better than uncrosslinked atelocollagen as an implant material in terms of volume retention and immunogenicity, in Frank DeLustro, "A comparative study of the biologic and immunologic response to medical devices derived from dermal collagen", Journal of Biomedical Materials Research, Vol.10, 20, 109-120 ( 1986 ); R. F. Oliver et al. "Histological studies of subcutaneous and intraperitoneal implants of tripsin-prepared dermal collagen allografts in the rats", Clin. Orthop Rel. Res. 115, 291-302 ( 1976 ) and M. J. Tavis, "Graft adherence to de-epithelialized surfaces ( A comparative study )", Ann. Surg., 184, 594-600 (1976).

However, the problem involved in this method is polymerizability of glutaraldehyde; when atelocollagen cross linked with glutaraldehyde is used as an implant material, polymeric glutaraldehyde contained therein tends to undergo polymerization in the living body though it advances slowly, thus exhibiting cytotoxicity.

It has thus been desired to provide an implant composition showing a high degree of volume retention and low immunogenicity in a living body and free of cytotoxicity.

It has also been desired to provide an implant composition highly effective in strengthening soft tissues and in the therapy and corrective therapy against congenital malformation and acquired defects.

According to the invention there is provided an implant composition comprising an aqueous dispersion of atelocollagen equilibrated with biological conditions, in which 60 to 100 weight % of the atelocollagen is crosslinked with hexamethylene diisocyanate.

In the implant composition of this invention, the proportion of crosslinked atelocollagen in the amount of total atelocollagen must be 60 weight % or larger to ensure intended high volume retention and low immunogenicity. The content of total atelocollagen in the composition should preferably be 25 to 100 mg/ml. The particle size of crosslinked atelocollagen should preferably be in the range of about 50 to 200 μm when the composition is to be injected into a living body through a fine injection syringe needle, but it is possible of course to implant dispersions of larger particle sizes by the use of a suitable injection device.

Also according to the present invention there is provided a method of preparing an implant composition which comprises an aqueous dispersion of atelocollagen equilibrated with biological conditions by a buffer solution, 60 to 100 weight % of said atelocollagen being crosslinked with hexamethylene diisocyanate, comprising:

(a) neutralizing an acidic aqueous solution of atelocollagen to give an aqueous dispersion containing regenerated atelocollagen fibers;

(b) dehydrating the aqueous dispersion by displacing water with alcohol to give an alcoholic dispersion containing regenerated atelocollagen fibers;

(c) crosslinking the regenerated atelocollagen fibers in the alcoholic dispersion with hexamethylene diisocyanate;

(d) removing unreacted hexamethylene diisocyanate by washing the alcoholic dispersion with a large amount of alcohol; and

(e) displacing alcohol in the alcoholic dispersion with water to give an aqueous dispersion, then adding an aqueous solution of uncrosslinked atelocollagen in such an amount that the proportion of uncrosslinked atelocollagen in the amount of total atelocollagen will be less than 40 weight %, and

2

subsequently equilibrating the resultant dispersion with biological conditions by addition of a buffer solution.

The degree of crosslinking of regenerated atelocollagen fibers with hexamethylene diisocyanate depends on reaction conditions adopted, particularly the concentration of the crosslinking agent used, the reaction temperature and time. Crosslinking reaction is usually carrid out in an alcohol (for example, ethanol) at room temperature for 0.5 to 72 hours. The amount of hexamethylene diisocyanate used is in the range of 0.5 to 500 mg for one gram of collagen (on dry basis), with its concentration in the reaction system being preferably in the range of 0.01 to 0.5%. After the reaction is allowed to proceed for a desired period of time, the reaction mixture was washed with a large amount of alcohol to terminate the reaction and to remove unreacted crosslinking agent at the same time. The resulting dispersion is then washed with water to displace alcohol therein with water, the aqueous dispersion thus obtained is concentrated in a centrifuge to a collagen concentration of about 30 to 120 mg/ml, and the concentrate is treated in a grinder ( for example, a homogenizer ) to grind the particles of crosslinked atelocollagen contained to sizes of 50 to 200 μm.

Finally, a phosphate buffer, for instance, selected from the combinations $KH_2PO_4$-$K_2HPO_4$, $KH_2PO_4$-$Na_2HPO_4$ and $NaH_2PO_4$-$Na_2HPO_4$ is added to equilibrate the aqueous dispersion with a biological condition and to adjust the concentration of total atelocollagen to 25 to 100 mg/ml.

As may be apparent from the foregoing, the implant composition of this invention comprising atelocollagen crosslinked with hexamethylene diisocyanate have high degree of volume retention and low immunogenicity, and are free of cytotoxicity. Because of these outstanding features, the implant compositions of this invention are very effective in strengthening soft tissues and in the therapy and corrective therapy against congenital malformations and acquired defects.

Example1

A portion of the skin was peeled off from the back of a calf, its periphery was cut out, and the remainder was washed with tap water to remove dirts and dusts from the surface and then washed with pyrogen-free water. The calf hide thus obtained was immersed in 70% ethanol, and all hairs and the uppermost surface of the skin were sliced off with a razor blade so as not to leave any hair roots. Care was taken during this operation to keep the exposed surface clean. The back surface was also treated in the same way, thus isolating a clean piece of calf dermis. This was kept immersed in 70% ethanol overnight, excess ethanol was removed, and the dermis was pulverized under germ-free con ditions. The pulverized dermis was then washed with a pyrogen-free, 5% aqueous NaCl solution, dehydrated by centrifugations, washed with pyrogen-free water, and again left immersed in 70% ethanol overnight. The excess alcohol was centrifugally removed, the resultant powder of dermis was put in a germ-free dissolver, pyrogen-free water was added, and a solution of pepsine with pyrogen-free water, freed from germs by filtration, was added. The amount of pepsine used was 0.5% of the dermis powder on dry basis. The concentration of the dermis powder was kept at about 0.5 to 0.9%, the pH value was adjusted to 3 by addition of hydrochloric acid, and the temperature was maintained at 20°C. The mixture in the dissolver was treated with mildly stirring for three days to completely dissolve collagen contained in the dermis, the solution of atelocollagen thus obtained was successively passed through filters having pore sizes 1μ, 0.65μ and 0.45μ in that order, and a caustic soda solution was added to the filtrate to raise the pH value to 11.0 to deactivate pepsine. The pH value was lowered to 7.0 by addition of hydrochloric acid to precipitate atelocollagen, which was collected by centrifugal separation. The atelocollagen thus obtained was washed with pyrogen-free water and dissolved in pyrogen-free hydrochloric acid of pH 3, and a caustic soda solution was added to raise the pH value to7.0 to precipitate regenerated atelocollagen fibers, thus giving its aqueous dispersion. Caustic soda was further added to pH 10.0 to activate amino groups in the atelocollagen, and the resulting dispersion was dehydrated by displacing water with ethanol until the ethanol concentration measured by an alcoholmeter exceeded 95 degrees. To an ethanolic dispersion containing 1% collagen thus obtained, was added hexamethylene diisocyanate in an amount of 300 mg per gram of collagen (on dry basis), and crosslinking reaction was conducted at 20°C for 20 hours. After the reaction is completed, the reaction mixture was washed with a large amount of ethanol to remove unreacted diisocyanate, ethanol was removed from the dispersion by displacement with water, and the aqueous dispersion thus obtained was concentrated by centrifugal separation to a collagen concentration of 40 mg/ml. The concentrate was then treated in a glass homogenizer to reduce the size of atelocollagen particles to about 50 to 100 μm. Crosslinking degree of the atelocollagen contained in the concentrate measured by TNBS method ( colorimetry using ) 2,4,6-trinitrobenzenesulfonic acid ) was 69%. Finally, a phosphate buffer ( 1M $NaH_2PO_4$-$Na_2HPO_4$) was added to

the homogenizer-treated dispersion, affording an implant composition containing 0.1M $NaH_2PO_4$-$Na_2HPO_4$ - (pH: 7.0; total atelocollagen concentration : 35 mg/ml).

An implantation test, a cytoxicity test and a test on stability against collagenase were conducted for the implant composition prepared above.

### 1) Implantation test

Skin tests for the implant composition obtained above were conducted for 504 patients who needed corrective therapy, and the positive response was observed with five persons ( 1.0% ). The rest of patients ( 499 persons ) were treated using the compositions, and allergic reaction ( erythema ) was observed with only three persons ( 0.6% ).

Similarly, skin tests were conducted for 608 patients using an aqueuos solution of uncrosslinked atelocollagen, and the positive response was observed with 24 persons (3.9%). The rest of patients ( 584 persons ) were treated using the solution, and allergic reaction ( erythema ) was obsereved with 11 persons ( 1.9% ).

As can be seen from the above results, the implant composition of this invention showed lowered immunogenicity compared with uncrosslinked atelocollagen.

### 2) Cytotoxicity test

An in-vitro culture test for human fibroblasts was carried out to examine the cytotoxicity of implant compositions containing atelocollagen. Various types of atelocollagen solutions were prepared, each was coated on a culture dish and air-dried, $1 \times 10^5$ fibroblasts were spread and cultivated for seven days, and the number of cells was counted and their shapes were observed. A culture dish with no collagen coated thereon was also tested as control. The types of atelocollagen used in the test and the result obtained are listed in the table below.

| Type of Atelocollagen | Number of Cells | Shapes of Cells |
|---|---|---|
| 1. Crosslinked with hexamethylene diisocyanate ( this invention ) | $8.4 \times 10^5$ | Normal |
| 2. Crosslinked with glutar-aldehyde | $2.7 \times 10^5$ | Very abnormal |
| 3. Uncrosslinked | $5.4 \times 10^5$ | Normal |
| 4. No collagen | $4.7 \times 10^5$ | Normal |

AS is apparent from the above table, no cytotoxicity was observed at all with the implant composition of this invention.

### 3) Test on stability against collagenase

An implant composition of this invention prepared above was treated with a collagenase of microbial origin in order to evaluate its volume retention in a living body. It was found that about 67.5% of the crosslinked atelocollagen contained was decomposed.

The same collagenase decomposed almost 100% of uncrosslinked atelocollagen under the same conditions.

This indicates that the crosslinked atelocollagen has higher stability against collagenase and hence shows a higher degree of volume retention in a living body than uncross linked atelocollagen.

Example 2

An implant composition was prepared in the same manner as that in Example 1, except that an aqueous solution of uncrosslinked atelocollagen was added to the concentrate containing particles of crosslinked atelocollagen 50 to 100 $\mu$m in size in such an amount that the proportion of uncrosslinked atelocollagen will be 20 weight % of total atelocollagen.

The implant composition thus obtained was subjected to a culture test and a cytotoxicity test in the same way as those in Example 1, and the similar results to those in Example 1 were obtained.

**Claims**

1. An implant composition comprising an aqueous dispersion of atelocollagen equilibrated with biological conditions by a buffer solution, 60 to 100 weight % of the atelocollagen being crosslinked with hexamethylene diisocyanate.

2. An implant composition according to claim 1, in which the amount of total atelocollagen in said dispersion is in the range of 25 to 100 mg/ml.

3. An implant composition according to claim 1 or 2, in which the particle size of crosslinked atelocollagen is in the range of 50 to 200 $\mu$m.

4. A method of preparing an implant composition which comprises an aqueous dispersion of atelocollagen equilibrated with biological conditions by a buffer solution, 60 to 100 weight % of said atelocollagen being crosslinked with hexamethylene diisocycanate, comprising

(a) dehydrating the aqueous solution of atelocollagen to give an aqueous dispersion containing regenerated atelocollagen fibers;

(b) dehydrating the aqueous dispersion by displacing water with alcohol to give an alcoholic dispersion containing regenerated atelocollagen fibers;

(c) crosslinking the regenerated atelocollagen fibers in the alcoholic dispersion with hexamethylene diisocyanate;

(d) removing unreacted hexamethylene diisocyanate by washing the alcoholic dispersion with a large amount of alcohol; and

(e) displacing alcohol in the alcoholic dispersion with water to give an aqueous dispersion, then adding an aqueous solution of uncrosslinked atelocollagen in such an amount that the proportion of uncrosslinked atelocollagen in the amount of total atelocollagen will be less than 40 weight %, and subsequently equilibrating the resultant dispersion with biological conditions by addition of a buffer solution.

5. A method according to claim 4, in which the amount of total atelocollagen in the aqueous dispersion equilibrated with biological conditions is in the range of 25 to 100 mg/ml.

6. A method according to claim 4 or 5, in which the particle size of crosslinked atelocollagen is in the range of 50 to 200 $\mu$m.